(19) **Europäisches Patentamt / European Patent Office / Office européen des brevets**

(11) **EP 2 956 798 B1**

(12) # EUROPEAN PATENT SPECIFICATION

(45) Date of publication and mention
of the grant of the patent:
**12.06.2019 Bulletin 2019/24**

(51) Int Cl.:
*G01S 15/89* (2006.01)   *A61B 8/06* (2006.01)

(21) Application number: **14706132.9**

(22) Date of filing: **29.01.2014**

(86) International application number:
**PCT/JP2014/052599**

(87) International publication number:
**WO 2014/125971 (21.08.2014 Gazette 2014/34)**

(54) **SUBJECT INFORMATION ACQUISITION APPARATUS, SUBJECT INFORMATION ACQUISITION METHOD, AND PROGRAM**

PERSONENINFORMATIONSERFASSUNGSVORRICHTUNG, PERSONENINFORMATIONSERFASSUNGSVERFAHREN UND PROGRAMM

APPAREIL D'ACQUISITION D'INFORMATIONS PAR SUJET, PROCÉDÉ D'ACQUISITION D'INFORMATIONS PAR SUJET, ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2013 JP 2013025728**
**18.11.2013 JP 2013238314**

(43) Date of publication of application:
**23.12.2015 Bulletin 2015/52**

(73) Proprietor: **Canon Kabushiki Kaisha**
**Tokyo 146-8501 (JP)**

(72) Inventors:
• **OKUMURA, Shigeaki**
  **Kyotp-shi, Kyoto 6068501 (JP)**
• **TAKI, Hirofumi**
  **Kyotp-shi, Kyoto 6068501 (JP)**
• **SATO, Toru**
  **Kyotp-shi, Kyoto 6068501 (JP)**
• **NAGAE, Kenichi**
  **Tokyo 146-8501 (JP)**

(74) Representative: **Foxon, Rachel Siobhan**
**Canon Europe Ltd**
**European Patent Department**
**3 The Square**
**Stockley Park**
**Uxbridge, Middlesex UB11 1ET (GB)**

(56) References cited:
**US-A- 5 718 229**

• ERIK GUDMUNDSON ET AL: "Blood velocity estimation using ultrasound and spectral iterative adaptive approaches", SIGNAL PROCESSING, ELSEVIER SCIENCE PUBLISHERS B.V. AMSTERDAM, NL, vol. 91, no. 5, 26 December 2010 (2010-12-26), pages 1275-1283, XP028131529, ISSN: 0165-1684, DOI: 10.1016/J.SIGPRO.2010.12.014 [retrieved on 2010-12-30]
• J-F SYNNEVAG ET AL: "Benefits of minimum-variance beamforming in medical ultrasound imaging", IEEE TRANSACTIONS ON ULTRASONICS, FERROELECTRICS AND FREQUENCY CONTROL, IEEE, US, vol. 56, no. 9, 1 September 2009 (2009-09-01), pages 1868-1879, XP011283394, ISSN: 0885-3010, DOI: 10.1109/TUFFC.2009.1263
• F. VIGNON ET AL: "Capon beamforming in medical ultrasound imaging with focused beams", IEEE TRANSACTIONS ON ULTRASONICS, FERROELECTRICS AND FREQUENCY CONTROL, vol. 55, no. 3, 1 March 2008 (2008-03-01), pages 619-628, XP055091879, ISSN: 0885-3010, DOI: 10.1109/TUFFC.2008.686
• TAKI H ET AL: "High Range Resolution Ultrasonographic Vascular Imaging Using Frequency Domain Interferometry With the Capon Method", IEEE TRANSACTIONS ON MEDICAL IMAGING, IEEE SERVICE CENTER, PISCATAWAY, NJ, US, vol. 31, no. 2, 1 February 2012 (2012-02-01), pages 417-429, XP011491032, ISSN: 0278-0062, DOI: 10.1109/TMI.2011.2170847

- **SASSO M ET AL: "medical ultrasound imaging using the fully adaptive beamformer", 2005 IEEE INTERNATIONAL CONFERENCE ON ACOUSTICS, SPEECH, AND SIGNAL PROCESSING - 18-23 MARCH 2005 - PHILADELPHIA, PA, USA, IEEE, PISCATAWAY, NJ, vol. 2, 18 March 2005 (2005-03-18), pages 489-492, XP010790683, DOI: 10.1109/ICASSP.2005.1415448 ISBN: 978-0-7803-8874-1**
- **VAITKUS P J ET AL: "A new time-domain narrowband velocity estimation technique for Doppler ultrasound flow imaging. I. Theory", IEEE TRANSACTIONS ON ULTRASONICS, FERROELECTRICS AND FREQUENCY CONTROL, IEEE, US, vol. 45, no. 4, 1 July 1998 (1998-07-01), pages 939-954, XP011437770, ISSN: 0885-3010, DOI: 10.1109/58.710565**
- **VAITKUS P J ET AL: "A new time-domain narrowband velocity estimation technique for Doppler ultrasound flow imaging. II. Comparative performance assessment", IEEE TRANSACTIONS ON ULTRASONICS, FERROELECTRICS AND FREQUENCY CONTROL, IEEE, US, vol. 45, no. 4, 1 July 1998 (1998-07-01), pages 955-971, XP011437771, ISSN: 0885-3010, DOI: 10.1109/58.710568**

## Description

### Technical Field

The present invention relates to a subject information acquisition apparatus, a subject information acquisition method, and a program, and in particular to a technique of transmitting acoustic waves to the subject, receiving reflected waves reflected in the subject, and thus acquiring movement information of an object in the subject.

### Background Art

An ultrasonograph, which is a subject information acquisition apparatus, has been widely used in a medical field and the like. The ultrasonograph can not only acquire morphology information reflecting a difference in an acoustic impedance in a living body, but can also acquire movement information of an object such as blood flow velocity information. In a pulse Doppler method, the movement velocity is calculated by detecting a phase shift of a reflected waveform from the object. For example, the blood flow velocity as movement information of the object is measured by detecting the phase shift of a received signal based on the reflected wave reflected from blood cells in a blood vessel. At that time, the reflected wave from the blood cells needs to be distinguished from a reflected wave (clutter component) from a reflector other than the measurement object, such as a surrounding blood vessel wall.

PTL 1 discusses a technique of using a moving target indicators (MTI) filter to distinguish the blood flow from a movement of a wall such as a blood vessel wall and a heart wall as the clutter component when a pulse Doppler method is used to calculate blood flow velocity. Three types of ultrasonic pulse transmission intervals are set. The difference between a received signal of a third pulse and a received signal of a first pulse (first differential signal), the difference between a received signal of a fourth pulse and a received signal of a second pulse (second differential signal), and the difference between a received signal of a fifth pulse and a received signal of the third pulse (third differential signal) are obtained. Then, the phase difference between the first and the second differential signals, and the phase difference between the second and the third differential signals are obtained. Then, the difference between the phase differences is further obtained.

ERIK GUDMUNDSON ET AL: "Blood velocity estimation using ultrasound and spectral iterative adaptive approaches", SIGNAL PROCESSING, ELSEVIER SCIENCE PUBLISHERS B.V. AMSTERDAM, NL, vol. 91, no. 5, 26 December 2010 (2010-12-26), pages 1275-1283, ISSN: 0165-1684, DOI: 10.1016/j.sigpro.2010.12.014 discloses iterative data-adaptive spectral estimation techniques for blood velocity estimation using medical ultrasound scanners. US 5,718, 229 discloses an ultrasonic imaging system that produces signals representative of tissue motion through amplitude detection of echo signals from which stationary clutter has been removed. A region to be imaged is insonified with ultrasonic energy. Two spatially aligned, temporally different echoes from each sample volume being imaged are applied to a stationary canceller or moving target indicator to remove stationary clutter. The received signals are amplitude detected and the detected signals undergo discrimination to distinguish moving tissue from blood flow and noise.

J-F SYNNEVAG ET AL: "Benefits of minimum-variance beamforming in medical ultrasound imaging", IEEE TRANSACTIONS ON ULTRASONICS, FERROELECTRICS AND FREQUENCY CONTROL, IEEE, US, vol. 56, no. 9, 1 September 2009 (2009-09-01), pages 1868-1879, ISSN: 0885-3010, DOI: 10.1109/TUFFC.2009.1263 discloses use of a minimum-variance beamformer to show how the low sidelobe levels and narrow beamwidth of adaptive methods can be used to enhance imaging in several ways.

### Citation List

#### Patent Literature

PTL 1: Japanese Patent Application Laid-Open No. 1-153144

### Summary of Invention

#### Technical Problem

As described above, when the blood flow velocity is measured, the phase shift of the signal based on the reflected waves from the blood cells is detected. However, the reflected wave from the blood cells is weaker than the reflected wave from the surrounding blood cell wall and the like. The detection of the phase shift of the received signal is susceptible to error when an unwanted signal (clutter signal) from the reflector other than the measurement object is included. The method of using the MTI filter as in PTL1 might seem like the solution, but such a filter might fail to sufficiently eliminate some types of clutter signals. The blood flow velocity is difficult to be accurately calculated especially when a clutter signal is large and when a reflector other than the measurement target object is fluctuating (moving) due to the beating of a tissue around the blood vessel, shaking of a hand of an operator, and the like.

In view of the problem described above, an object of the present invention is to acquire movement information of an object with a higher accuracy even under the presence of unwanted reflected waves in addition to the reflected waves from the target object.

**Solution to Problem**

According to an aspect of the present invention, there is provided a subject information acquisition apparatus as set out in claim 1.

According to an aspect of the present invention, there is provided a subject information acquisition method as set out in claim 8.

Further features of the present invention will become apparent from the following description of exemplary embodiments with reference to the attached drawings.

**Brief Description of Drawings**

Fig. 1 is a schematic diagram illustrating an overview of a subject information acquisition apparatus of Example 1.
Fig. 2 is a flowchart illustrating a processing flow in Example 1.
Fig. 3A and 3B are each a schematic diagram illustrating an example of a filter of the subject information acquisition apparatus of Example 1.
Fig. 4 is a schematic diagram illustrating an example of a display of the subject information acquisition apparatus of Example 1.
Fig. 5 is a schematic diagram illustrating a model for verifying an effect of Example 1.
Fig. 6 is a diagram illustrating an effect of Example 1.
Fig. 7 is a schematic diagram illustrating an overview of a subject information acquisition apparatus of Example 2.
Fig. 8 is a diagram illustrating compensation for a differential filter.

**Description of Embodiments**

Embodiments of the present invention will be described with reference to the drawings. As a general rule, components common among the embodiments are denoted with the same reference numerals and will not be repeatedly described.

In the present invention, an acoustic wave is typically an ultrasonic wave, and includes an elastic wave known as a sound wave and an ultrasonic wave. A subject information acquisition apparatus according to the present invention includes an apparatus that transmits acoustic waves to a subject, receives reflected waves reflected in the subject (reflected acoustic waves), and acquires information of the inside of the subject as a numeric value and image data. The information of the inside of the subject thus acquired includes movement information of an object, information reflecting a difference in an acoustic impedance of a tissue, and the like. The "movement information" of the object in the subject is information on "velocity" and "displacement". Specifically such information includes a blood flow velocity (movement information of a scatterer group including red blood cells and the like), a displacement of a tissue, and the like. The velocity and the displacement may be acquired as a velocity distribution and a displacement distribution.

**Example 1**

An apparatus configuration and a processing flow of Example 1 will be described below.

(Configuration of Subject Information Acquisition Apparatus)

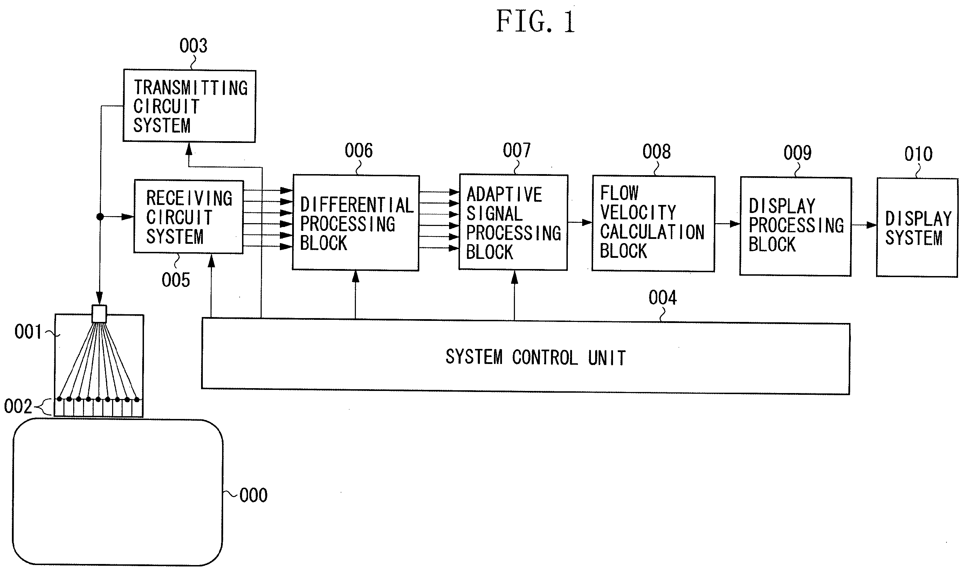

Fig. 1 is a schematic diagram illustrating a configuration of a subject information acquisition apparatus of Example 1. The subject information acquisition apparatus of the present embodiment includes a probe 001 including a plurality of transducers 002, a receiving circuit system 005, a transmitting circuit system 003, a differential processing block 006, an adaptive signal processing block 007, and a flow velocity calculation block 008. The subject information acquisition apparatus further includes a system control unit 004, a display processing block 009, and a display system 010.

The probe 001 is a receiver transmitter that transmits acoustic waves to a subject 000, and receives reflected waves reflected from a plurality of positions in the subject. The probe 001 includes the plurality of transducers 002 that convert acoustic waves into electrical signals (time series received signals). Although not illustrated in the subject 000, the subject includes a movement information measurement target object (for example, blood cells and moving tissues in the blood). The transducers 002 receive the reflected waves from the target object. The transducer 002 may be a transducer such as a piezoelectric element utilizing piezoelectric phenomenon, a transducer utilizing optical resonance, a transducer utilizing changes in capacitance such as a capacitive micromachined ultrasonic transducers (CMUT), or any other transducer that can receive an acoustic wave and convert the received acoustic wave into an electric signal. The transducers are preferably arranged in a form of an array such as a one dimensional (1D) array and a two dimensional (2D) array.

The transmission circuit system 003 is a transmission signal generator that generates a transmission signal (pulse signal) in accordance with a control signal from the system control unit 004. A voltage waveform of the transmission signal has a delay time and amplitude corresponding to a target position and a target direction. The transmission signal is input to each of the transducers 002 to be converted into an acoustic wave, and is transmitted as an acoustic wave pulse (pulse wave). The transducers 002 receive the reflected waves from the subject, and output a plurality of received signals to the receiving circuit system 005.

The receiving circuit system 005 includes an amplifier, an analog to digital (A/D) converter, and the like. The received signals output in time series from each transducer are amplified and converted into a digital signal (digitized received signal) by the receiving circuit system 005. The receiving circuit system 005 treats time series received signals output from a single transducer by a single acoustic wave transmission, as a single received signal. When the number of output channels of the transducers is M, M received signals corresponding to the number of the output channels are obtained by a single acoustic wave pulse transmission. Considering a single transducer, when the acoustic wave pulse transmission is performed for N times, the received signals corresponding to the N times (that is, N time series received signals) are obtained from each transducer. Each of N and M are a positive integer.

In the present invention, not only an analog received signal output from the transducer 002, but also a signal obtained by the amplification, the digital conversion, and the like on the received signal is also referred to as the received signal. The received signal output from each of the output channels of the receiving circuit system 005 is input to the differential processing block 006. The output channels of the receiving circuit system 005 correspond to the output channels of the transducers. However, the number of the transducers may not necessarily be the same as the number of the output channels. The number of the transducers may be smaller than the number of the output channels.

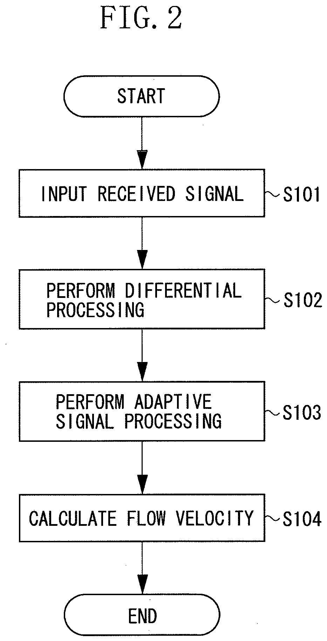

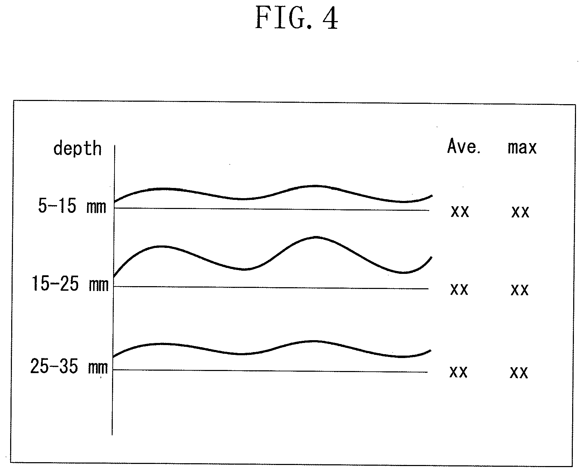

The differential processing block 006 is a differential processing unit that acquires a differential signal, and includes a differential filter such as a MTI filter. The differential processing block 006 uses a plurality of received signals obtained by transmitting the acoustic wave pulse for a plurality of times to calculate a differential signal representing a difference between the received signals, for each output channel. The processing performed by the differential processing block 006 will be described later in detail with reference to step S102 in Fig. 2 and Fig. 4.

The adaptive signal processing block 007 is an adaptive signal processing unit that uses the differential signals output from the differential processing block 006 to perform adaptive signal processing. The adaptive signal processing corresponds to an adaptive beam forming. More specifically, the adaptive signal processing refers to processing of adaptively changing a processing parameter such as a phase and a weight in accordance with a received signal to selectively extract a received signal based on a desired wave as a target in a target direction or from a target position, and reducing other received signals based on unwanted waves. Typically, the CAPON method that is one type of the adaptive signal processing is a method of minimizing an output (power strength) for a plurality of input signals with a fixed sensitivity related to the target direction and the target position. The CAPON method is also referred to as Directionally Constrained Minimization of Power (DCMP) and Minimum variance method. Such adaptive signal processing has an effect of improving a spatial resolution. An example where the CAPON method is used as the adaptive signal processing in step S103 in Fig. 2 will be described in detail. Instead of the CAPON method used in the present embodiment, another adaptive signal processing (MUSIC method and ESPRIT method) may be used.

The flow velocity calculation block 008 uses an output signal output from the adaptive signal processing block 007 to calculate movement information on an object such as a blood flow velocity. For example, a velocity at a single point (a single position) in the object, or velocities at a plurality of positions in a depth direction may be obtained as the flow velocity. Alternatively, an average velocity or a maximum velocity in a predetermined depth range may be obtained. Still alternatively, velocities at a plurality of time series points may be obtained so that how the speed changes over time can be displayed.

In the present embodiment, a processing unit at least includes the receiving circuit system 005, the differential processing block 006, the adaptive signal processing block 007, and the flow velocity calculation block 008. The processing unit of the present embodiment may further include a delay-and-sum block and an envelope detection processing block (none of which are illustrated in Fig. 1). With the delay-and-sum block and the envelope detection processing block, a distribution (acoustic characteristic distribution) reflecting a difference in an acoustic impedance such as a B mode image can be acquired in addition to the movement information.

The display processing block 009 uses the input movement information to generate display data, and outputs the display data to the display system 010. More specifically, the display processing block 009 processes the input movement information to generate image data for displaying a value of a velocity, a graph illustrating how the velocity changes over time, a velocity distribution indicating velocities at a plurality of positions, and the like. When the processing unit is able to also acquire the acoustic characteristic distribution, the display processing block 009 may generate image data for displaying the movement information and the acoustic characteristic distribution side by side on a single display screen, and image data for displaying the velocity distribution obtained as the movement information and the acoustic characteristic distribution in a superimposed manner. A display mode can be changed by an instruction from the system control unit 004 that has received an input from a user as an operator.

In the present embodiment, the differential processing block 006, the adaptive signal processing block 007, the flow velocity calculation block 008, the display processing block 009, and the system control unit 004 implemented by processing devices such as a central processing unit (CPU), a graphic processing unit (GPU), and a field programmable gate array (FPGA) chip.

The display system 010 is a display device that displays an image based on the display data output from the display processing block 009, and is a liquid crystal display (LCD), a cathode ray tube (CRT), an organic light emitting display, or the like. The display unit 010 may not be provided in the subject information acquisition apparatus of the present invention. Instead, the display system 010 may be separately provided and connected to the subject information acquisition apparatus.

(Processing Flow of Processing Unit)

Next, a processing flow of the processing unit will be described with reference to Fig. 2. Fig. 2 is a flowchart illustrating the processing flow of Example 1.

First, in step S101, a time series received signal from each output channel of the receiving circuit system 005 is input to the differential processing block 006. A signal $x_{k,l}[s]$ represents an l-th received signal obtained by an l-th acoustic wave transmission and output from a k-th (k=1, 2, ..., M) output channel of the receiving circuit system 005. The sign "s" means that the signal is of an s-th sample of a single time series received signal (that is, one point in the time series) .

Next, in step S102, the differential processing block 006 calculates the difference between two input received signals for each output channel (for example, a difference between the received signals respectively obtained by the first and the second acoustic wave pulses) using the following Formula (1), and outputs a differential signal $d_{k,l}[s]$:

$$d_{k,l}[s] = x_{k,l+1}[s] - x_{k,l}[s] \ \ldots \ \text{Formula (1)}.$$

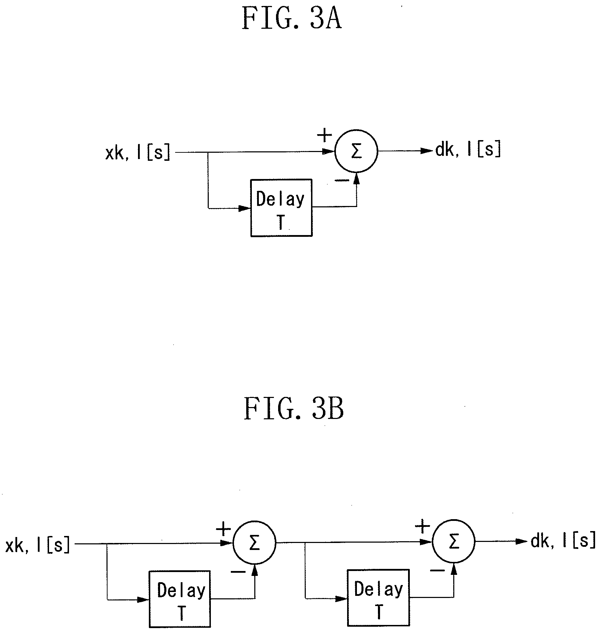

Fig. 3A is a schematic diagram illustrating example differential filters used for the differential processing using a differential filter. A time period corresponding to a transmission interval between acoustic wave pulses (time interval between (l+1)-th transmission and l-th transmission) is set as Delay T. Thus, the difference between $X_{k,l+1}[s]$ and $x_{k,l}[s]$ is output. The differential processing block 006 extracts a plurality of differential signals through the processing described above for each output channel, and inputs the differential signals to the adaptive signal processing block 007. In the configuration illustrated in Fig. 3A, at least two differential signals are required for each output channel. The differential filter used in the present embodiment is not limited to the differential filter illustrated in Fig. 3A that calculates the difference between the two received signals, and a differential filter having a configuration illustrated in Fig. 3B may be used.

In step S103, the adaptive signal processing block 007 uses a plurality of input differential signals to perform the adaptive signal processing. The number of the differential signals is the same as the number of the output channels. In Example 1, processing using the CAPON method is described as the adaptive signal processing.

The adaptive signal processing block 007 first uses information of a target position (a predetermined position in the subject) instructed from the system control unit 004 to perform what is called phasing processing. Specifically, delay processing is performed to synchronize the phases of the received signals corresponding to the target position. Hilbert transform is performed on the signal thus obtained by the phasing processing. A complex representation of the signal thus obtained is defined as $x'_{k,l}[s']$. An input vector $X_l[s']$ of an s'-th sample is defined as follows with the signal.

$$X_l'[s'] = \left[ x'_{1,l}[s'], x'_{2,l}[s'], \cdots, x'_{M,l}[s'] \right]^T \qquad \text{[Formula 1]}$$

The adaptive signal processing block 007 uses the input vector $X_l[s']$ to calculate a relative matrix $R_{xx,l}$.

$$R_{xx,l} = E\left[X_l[s']X_l^H[s']\right]$$

$$= \begin{bmatrix} E\left[x_{1,l}[s']x_{1,l}^*[s']\right] & E\left[x_{1,l}[s']x_{2,l}^*[s']\right] & \cdots & E\left[x_{1,l}[s']x_{M,l}^*[s']\right] \\ E\left[x_{2,l}[s']x_{1,l}^*[s']\right] & E\left[x_{2,l}[s']x_{2,l}^*[s']\right] & \cdots & E\left[x_{2,l}[s']x_{M,l}^*[s']\right] \\ \vdots & \vdots & \ddots & \vdots \\ E\left[x_{M,l}[s']x_{1,l}^*[s']\right] & E\left[x_{M,l}[s']x_{2,l}^*[s']\right] & \cdots & E\left[x_{M,l}[s']x_{M,l}^*[s']\right] \end{bmatrix}$$

[Formula 2]

In Formula 2, the superscript H represents complex conjugate transposition, and the superscript * represents complex conjugate. E[·] represents processing to calculate a time average. Specifically, the sample number (s' herein) is varied, and the average value is calculated. Thus, this is processing to average the relative matrix calculated by using the input vector in the depth direction. The depth direction corresponds to a traveling direction of the acoustic wave (ultrasound wave beam) transmitted from the probe. The time average range preferably does not exceed the resolution in a predetermined depth, and differ among observed depths. Instead of the CAPON method involving no spatial averaging used in the present embodiment, a CAPON method involving the spatial averaging may be used.

Next, the adaptive signal processing block 007 obtains a weight vector under the conditions of the following Formula 3:

$$\min_W (W^H R_{xx,l} W)$$

$$\text{subject to } W^H a = 1$$  [Formula 3].

The conditions mean that the output (power strength) is minimized with the sensitivity in a desired direction (target direction) fixed to 1. In the formula, "a" represents a steering vector that defines the desired direction, that is, an observation direction. An optimum weight Wopt is calculated under such conditions as follows:

$$Wopt = \frac{(R_{xx,l} + \eta E)^{-1} a}{a^H (R_{xx,l} + \eta E)^{-1} a}$$  [Formula 4].

In the formula, $\eta E$ is added to stabilize the inverse matrix calculation, $\eta$ is a constant or a value changing in accordance with the value of $R_{xx,l}$ and the like, and "E" is an identity matrix. With the optimum weight, the power output can be minimized with the sensitivity in the desired direction set to 1. With the optimum weight, a reception pattern having such directionality that the sensitivity in the desired direction is 1 and the sensitivity in an arrival direction of a noise component is low can be obtained. The adaptive signal processing block 007 multiplies the optimum weight Wopt by the input vector Xl[s'] to obtain an output signal $y_l[s']$.

$$y_l[s'] = W^H opt X_l'[s']$$  [Formula 5]

In step S104, the flow velocity calculation block 008 uses $y_l[s']$ output from the adaptive signal processing block 007 to calculate a flow velocity $v_c$ as the movement information through Formula 6:

$$v_c = \frac{\lambda_c \overline{\theta_c[s']}}{4\pi T}$$

$$= \frac{\lambda_c \arg\left[\int_{S_{obs}-S_{ave}/2}^{S_{obs}+S_{ave}/2} y_l[s']^* y_{l+1}[s']ds'\right]}{4\pi T}$$  [Formula 6].

In the formula, $S_{obs}$ represents a sample number corresponding to an observed depth (predetermined position in a depth direction), $S_{ave}$ represents the time average range, T represents a repetition period of the acoustic wave pulse transmission (time interval between (l+1)-th transmission and 1-th transmission), $\lambda_c$ represents the wavelength of the transmission frequency, and $\theta_c$ represents the phase difference between $y_l$ and $y_{l+1}$. Thus, the phase difference between the differential signals (phase shift) obtained by the repetitive transmission and reception is calculated and used to obtain the movement velocity of the measurement object. A displacement can be calculated by multiplying the movement velocity by the repetition period.

The flow velocity $v_c$ thus calculated is input to the display processing block 009. The display processing block 009 uses the input flow velocity to generate image data as described above, and outputs the image data to the display system 010.

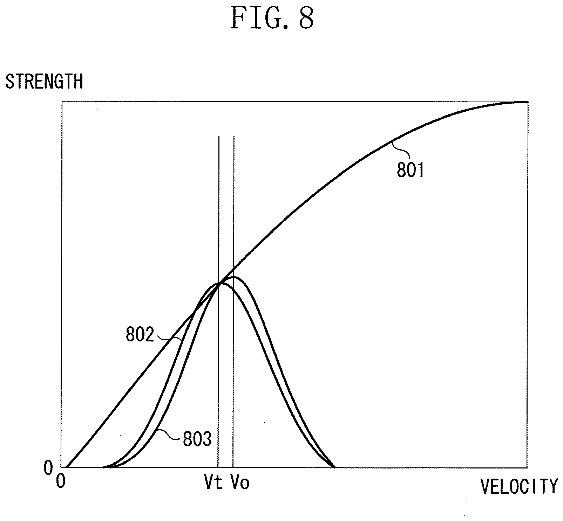

Now, processing of a compensation for a differential filter will be described. Fig. 8 is a diagram illustrating the compensation for the differential filter. The differential filter reduces a low-velocity signal to reduce low frequency components. For example, a graph 801 schematically illustrates an example of an influence of the differential filter on a velocity. A broken line 803 in the figure schematically illustrates a velocity distribution obtained by calculating a velocity for a plurality of times through the processing described above, and an average velocity Vo is calculated from the velocity distribution. However, the velocity distribution 803 is affected by the differential filter 801, and an actual velocity distribution is the one schematically represented by a solid line 802. Therefore, the average velocity (Vo) might be calculated as a value higher than an actual average velocity Vt due to the influence of the differential filter.

In the present embodiment, an inverse filter for eliminating the influence of the differential filter is used for the velocity distribution obtained by a plurality of times of measurement to newly calculate a velocity distribution. An average value or a median of the velocity distribution may be used to estimate a corrected flow velocity. A value of the corrected flow velocity may be input to the display processing block 009 as a calculated flow velocity vc.

Thus, an apparatus can be provided that performs the compensation for the differential filter, and thus is able to estimate the flow velocity more accurately.

Now, an example of an image displayed on the display system 010 is described with reference to Fig. 4. Fig. 4 illustrates how the flow velocity changes over time in three types of depth ranges (5 to 15 mm, 15 to 25 mm, and 25 to 35 mm). The average flow velocity and the maximum flow velocity in each depth range are displayed as numerical values.

(Effect of Present embodiment)

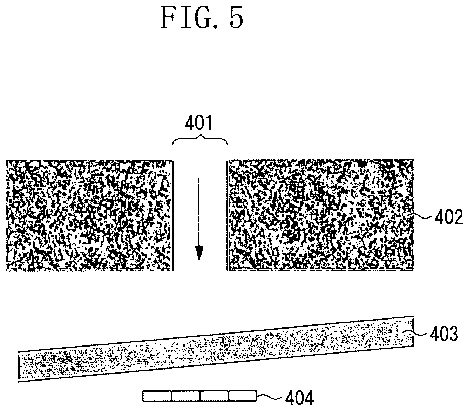

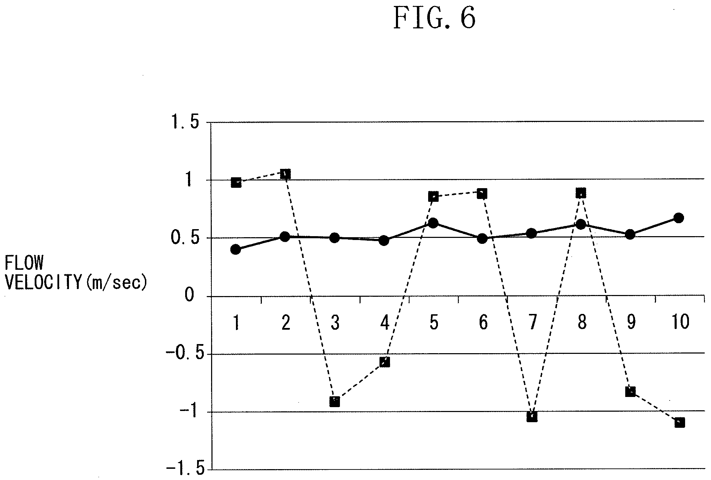

Next, an effect of the present embodiment will be described with reference to Fig. 5 and Fig. 6. Fig. 5 is a simple model of a living body. In the model, the blood flows in a direction of an arrow in an area 401, and a surrounding tissue 402 is provided around the area 401. A strong reflector 403 is disposed in a path through which ultrasonic waves as the acoustic waves are transmitted and received. The strong reflector 403 is assumed to be a skull bone and the like. In this simulation, the ultrasonic waves are transmitted from four transducers 404, and reflected waves from a portion around the area 401 are acquired. The processing of the present embodiment is performed with an input signal obtained by providing a time-varying fluctuation to the acquired received signal. The fluctuation is assumed as a body motion such as beating and hand shaking of a user operating the probe. Results of the simulation are illustrated in Fig. 6.

The horizontal axis in Fig. 6 represents processing results of cases with different fluctuations, and the vertical axis represents the calculated flow velocity. In the model, an approaching velocity of the blood to the probe is set to be 0.5 m/sec, and thus +0.5 m/sec is the true value. In Fig. 6, the dotted line represents results of calculating the flow velocity only by the differential processing in the differential processing block 006. In other words, the dotted line represents flow velocities obtained when the differential signals obtained by the differential processing block 006 are directly input to the flow velocity calculation block 008. As can be seen in the results, the direction of the blood flow and the absolute value of the velocity of the blood flow largely differ from the true value.

The solid line in Fig. 6 represents results obtained by the present embodiment. Specifically, the solid line represents the results obtained by calculating the flow velocities by the flow velocity calculation block 008 using the output from the adaptive signal processing block 007. It can be seen in the figure that the difference between the results illustrated in the solid line and the true flow velocity 0.5 m/sec is small. Thus, the blood flow velocity is obtained with higher accuracy. Thus, in the present embodiment, a velocity can be accurately obtained even under the presence of a large clutter component, and even when the reflector as the clutter component is fluctuating (moving).

Next, the reason why the effect of the present embodiment is obtained will be described. The differential processing is processing of using a plurality of time series received signals to extract a signal representing a time varying component between the received signals as a signal representing a component (movement component) based on the reflected wave from the reflector moving in the subject. In other words, the function of the differential processing is to reduce (ideally, eliminate) the received signals based on the reflected waves from the reflector that stays still during the repetition period T as the pulse transmission interval. Still, the differential processing, which eliminates the signal from a reflector that remains still during a repetition period T, might fail to completely eliminate a signal (clutter) of a clutter component from a moving reflector. The results represented by the dotted line in Fig. 6 represent a case where such clutter signals failed to be eliminated remain and affect the flow velocity estimation.

In the present embodiment, even when such clutter signals failed to be eliminated by the differentiation processing are included, the clutter components from directions other than the target direction (desired direction) can be further reduced. Thus, signals based on the reflected waves from the object can be selectively acquired with the clutter signals of the reflected waves from the moving reflector other than the object being reduced. Thus, a flow velocity can be more accurately estimated.

In the present embodiment, ideally, all the components that do not vary over time between received signals (time invariant component) are eliminated, and the components that vary over time (time varying component) between received signals are mainly acquired. However, actually, a differential filter such as the MTI filter used as the differential processing block 006 might fail to completely eliminate the time invariant components. Furthermore, the differential filter might even partially eliminate the time varying components with small time variance (i.e., low frequency component with slow movement).

In the present invention, the term "signal representing the time varying component is extracted" includes not only a case where all the time varying components are extracted but also includes a case where the time varying components are at least partially extracted. Furthermore, the time invariant components may be partially included in addition to the time varying components. The differential processing is effective even when not all the time invariant components are eliminated, as long as the time invariant components are reduced compared with the case where the differential processing is not performed.

Similarly, in the present invention, "signal representing a movement component is extracted" includes not only a case where all the signals based on the reflected waves from the moving reflector are extracted, but also includes a case where the signals based on the reflected waves from the moving reflector are at least partially extracted. Furthermore, signals based on the reflected waves from the unmoving reflector may be partially included in addition to the signal representing the movement component. The processing in each of the first to the third exemplary embodiments is effective as long as the signals based on the reflected waves from an unmoving reflector are reduced.

The "signal representing time varying component" thus extracted is a signal suitable for the adaptive signal processing. The basic operation in the adaptive signal processing is to reduce the clutter components arriving from a predetermined direction. In the present embodiment, the time invariant components in the extracted "signal representing the time varying component between the received signals" are reduced. Thus, the power for the clutter components is reduced in advance. Thus, the adaptive signal processing operates effectively by using the "signal representing the time varying components". In other words, the time varying components of an object can be calculated with the electric power caused by the clutter components in directions failed to be eliminated by the adaptive signal processing being reduced (i.e., the time varying components of the clutter signals can be reduced).

**Example 2**

Next, Example 2 will be described. A feature of Example 2 is that a quadrature detection and low-pass filter processing are performed on a received signal, and high-pass filter processing is applied to the resultant signal to extract a signal representing a time varying component. In the present embodiment, a distribution (acoustic characteristic distribution) reflecting the difference in an acoustic impedance such as a B mode image can be acquired in addition to the movement information. The point different from Example 1 will be described below.

(Configuration of Subject Information Acquisition Apparatus)

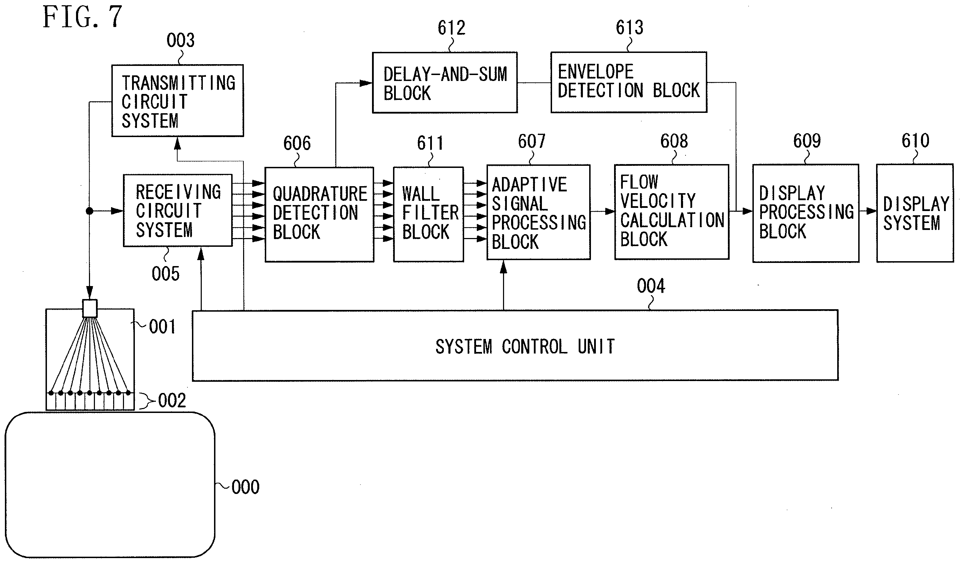

Fig. 7 is a schematic diagram illustrating a configuration of a subject information acquisition apparatus according to the present embodiment. The subject information acquisition apparatus according to the present embodiment includes the probe 001 including the plurality of transducers 002, the receiving circuit system 005, the transmitting circuit system 003, a quadrature detection block 606, a wall filter block 611, an adaptive signal processing block 607, and a flow velocity calculation block 608. The subject information acquisition apparatus further includes the system control unit 004, a display processing block 609, a delay-and-sum block 612, an envelope detection block 613, and a display system 610.

The probe 001 including the transducers 002, the transmitting circuit system 003, and the receiving circuit system 005 are the same as those described in Example 1, and thus will not be described.

The quadrature detection block 606 is a quadrature detection unit that performs quadrature detection processing on a time series received signal input from each output channel of the receiving circuit system 005. More specifically, the quadrature detection block 606 uses a signal of a waveform having a center frequency of the acoustic wave transmitted or received and a signal of a waveform obtained by shifting the phase of the signal by 90° as reference signals, and multiplies each received signal by the two reference signals. Thus, a quadrature detection is performed, and a quadrature detection signal in a complex representation is obtained. Then, the low-pass filter having a cutoff frequency that is approximately the same as the center frequency of the transmitted or received acoustic wave is applied to the calculated quadrature detection signal. Thus, the quadrature detection signal, from which unwanted harmonic components are eliminated, is obtained. The quadrature detection signal thus obtained for each output channel is input to the wall filter block 611.

The wall filter block 611 is a high-pass filter having high pass characteristics set through an instruction from the system control unit 004. The wall filter block 611 processes the quadrature detection signals obtained by a plurality of times of transmission and reception. The high pass filter can reduce the reflected wave components from an unmoving or slowly moving object.

The adaptive signal processing block 607 receives a plurality of signals output from each of the output channels of the wall filter block 611, and performs the adaptive signal processing. The signal output from the adaptive signal processing block 607 is input to the flow velocity calculation block 608, and the flow velocity calculation block 608 calculates the movement information of an object such as a blood flow velocity. The processing in the adaptive signal processing block 607 is the same as the processing in the adaptive signal processing block 007 in Example 1 except that the Hilbert transformation is omitted. The processing in the flow velocity calculation block 608 is the same as the processing in the flow velocity calculation block 008 in Example 1 and thus will not be described.

The quadrature detection signals output from the quadrature detection block 606 are also input to the delay-and-sum block 612. The delay-and-sum block 612 uses the received quadrature detection signals of each output channel to perform delay-and-sum processing in accordance with the target position. The delay-and-sum processing is processing of performing delay processing (phasing processing) in accordance with a distance between each element and the target position, and then performing addition processing. The resultant signal obtained by the delay-and-sum processing is subjected to filter processing if needed, and then is input to the envelope detection block 613. The envelope detection block 613 acquires an envelope corresponding to the resultant signal obtained by the delay-and-sum processing thus received, and obtains a distribution (acoustic characteristic distribution) reflecting a difference in an acoustic impedance such as a B mode image.

The display processing block 609 uses the movement information received from the flow velocity calculation block 608 and the acoustic characteristic distribution received from the envelope detection block 613 to generate display data. More specifically, display data for displaying an image illustrating movement information of each depth as illustrated in Fig. 4 and the B mode image illustrating the acoustic characteristic distribution side by side on the same display screen can be generated. Alternatively, display data for displaying an image illustrating a movement information distribution such as a velocity distribution indicating a velocity at each position and the B mode image indicating the acoustic characteristic distribution in a superimposed manner may be generated. The display mode can be changed by an instruction from the system control unit 004 that has received an input from the user as an operator. The display unit 610 displays an image based on the display data output from the display processing block 609.

The processing flow from the input of the received signal to the flow velocity calculation in the processing unit in the present embodiment is the same as that in Fig. 2 described in Example 1 except for step S102. In the present embodiment, the quadrature detection, the low-pass filter processing, and the high-pass filter processing are performed as the processing in step S102 instead of the differential processing.

More specifically, the unwanted harmonic components are eliminated by the low-pass filter processing after the quadrature detection. Then, the frequency components based on the reflected waves from a reflector with a small movement amount are reduced (ideally eliminated) by the high-pass filtering. Thus, the frequency components based on the reflected waves from a reflector with a large movement amount are extracted (moving in a higher velocity than a predetermined velocity). It is to be noted that the extracted frequency components at this point include frequency components based on the clutter components from an entity other than the object

Thus, the signals as the clutter components failed to be eliminated by the high-pass filtering processing are reduced by the adaptive signal processing. Thus, also in the present embodiment, the signal representing a time varying component between received signals can be extracted by the processing before the adaptive signal processing, and the signals as the clutter components failed to be eliminated by the adaptive signal processing can be reduced. Thus, more accurate and thus favorable velocity estimation can be achieved also in the present embodiment.

The system control unit 004 sets the high-frequency-side cutoff frequency of the low-pass filter in the quadrature detection block 606 and the low-frequency-side cutoff frequency of the wall filter block 611. The cutoff frequency of the low-pass filter in the quadrature detection block 606 is preferably equal to or lower than the center frequency of the transmitted or received acoustic wave. Preferably, the cutoff frequency of the wall filter block 611 is appropriately set in accordance with the movement velocity of the measurement object.

In the present embodiment, the distribution reflecting a difference in an acoustic impedance such as a B mode image (acoustic characteristic distribution) can be obtained in addition to the movement information. By thus acquiring the acoustic characteristic distribution, the movement information and the acoustic characteristic distribution can be displayed side by side or in a superimposed manner. Thus, the user can more easily recognize the association between the position in a subject and the blood flow velocity, whereby the usability is improved. It is a matter of course that the acoustic characteristic distribution can be acquired and displayed also in Example 1.

**Example 3**

In Example 1 and Example 2, the adaptive signal processing is performed after the signal representing the time varying component is extracted. In Example 3, the signal representing the time varying component is extracted after the adaptive signal processing is performed.

The apparatus configuration is different from those of the subject information acquisition apparatuses illustrated in Fig. 1 and Fig. 7 in the position of the adaptive signal processing block 007. Specifically, in Fig. 1, the adaptive signal processing block 007 is provided between the receiving circuit system 005 and the differential processing block 006. In Fig. 7, the adaptive signal processing block 607 is provided between the quadrature detection block 606 and the wall filter block 611.

In the present embodiment, a plurality of received signals input from a plurality of output channels are used to perform the adaptive signal processing. Then, signals representing the time varying component are extracted from the output signal obtained by the adaptive signal processing. The signal representing the time varying component is extracted in the same way as that described in Example 1 and Example 2. Then, a plurality of the signals representing the time varying component are used to acquire the movement information of an object.

A flow velocity can be estimated with a higher accuracy compared with a conventional case, by using the signal that has passed through differential filter and the wall filter after the adaptive signal processing according to the present embodiment. Still, the basic function of the adaptive signal processing is to reduce the clutter components arriving from a certain direction, from input signals as described in Example 1. This means that the signals from a reflector that remains still during the repetition period T are difficult to completely eliminate. Thus, the present invention is more effective when the adaptive signal processing is applied after the signals from unmoving reflectors are reduced, as in Example 1 and Example 2.

Other Embodiments

Embodiments of the present invention may be realized through the following processing. Specifically, the exemplary embodiments may be realized by the processing of supplying software (program) for realizing the functions of the exemplary embodiments described above to a system or an apparatus through a network or various storage media, and reading and executing the program by a computer (or a CPU, MPU, and the like) of the system or the apparatus.

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment (s) of the present invention, and by a method performed by the computer of the system or apparatus by, for example, reading out and executing the computer executable instructions from the storage medium to perform the functions of one or more of the above-described embodiment (s) . The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

According to the present invention, the movement information of an object can be acquired with a higher accuracy even under the presence of unwanted reflected waves.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. A subject information acquisition apparatus configured to acquire movement information of an object in a subject, the subject information acquisition apparatus comprising:

a plurality of transducers (002) configured to transmit an acoustic wave pulse to the subject, receive reflected waves reflected from a plurality of positions in the subject, each of the plurality of transducers coupled to a corresponding output channel, and convert the reflected waves respectively into time series received signals; and

a processing unit (006, 007, 008) configured to acquire the movement information of the object by using a plurality of the received signals output from the plurality of transducers via the corresponding output channels, **characterized in that** the processing unit extracts, for each of the output channels, a plurality of signals each representing a movement component and reduces, for each of the output channels, signals based on reflected waves from an unmoving reflector by using the plurality of received signals, performs adaptive beam forming by using the plurality of signals each representing the movement component, and acquires the movement information of the object by using a plurality of output signals obtained by the adaptive beam forming.

2. The subject information acquisition apparatus according to claim 1, wherein the processing unit extracts, for each of the output channels, a plurality of signals each representing a time varying component between received signals obtained by transmitting the acoustic wave pulse a plurality of times as the signals each representing the movement component.

3. The subject information acquisition apparatus according to claim 2, wherein the processing unit extracts the plurality of signals each representing the time varying component between the plurality of received signals, by obtaining, for each of the output channels, a difference between the plurality of received signals obtained by transmitting the acoustic wave pulse transmission a plurality of times.

4. The subject information acquisition apparatus according to claim 2, wherein the processing unit extracts the plurality of signals each representing the movement component by performing, for each of the output channels, low-pass filter processing and high-pass filter processing, after performing a quadrature detection on the plurality of received signals obtained by transmitting the acoustic wave pulse for a plurality of times.

5. The subject information acquisition apparatus according to any one of claims 1 to 4, wherein the processing unit extracts as the signal representing the movement component, a signal based on a reflected wave from a reflector moving in the subject at a higher velocity than a predetermined velocity.

6. The subject information acquisition apparatus according to any one of claims 1 to 5, wherein the processing unit performs, as the adaptive beam forming, processing of minimizing electric power with a sensitivity related to a target direction being fixed, by using the plurality of signals each representing the movement component.

7. The subject information acquisition apparatus according to any one of claims 1 to 6, wherein the processing unit further acquires an acoustic characteristic distribution reflecting a difference in an acoustic impedance in the subject, and generates display data by using the movement information and the acoustic characteristic distribution.

8. The subject information acquisition apparatus according to claim 3, wherein the processing unit further performs a compensation processing for reducing an effect of taking the difference.

9. A subject information acquisition method of acquiring movement information of an object in a subject by using a plurality of time series received signals output from a plurality of transducers that transmit an acoustic wave pulse to the subject and receive reflected waves reflected from a plurality of positions in the subject, each of the plurality of transducers coupled to a corresponding output channel, the subject information acquisition method comprising:

extracting (S102), for each of the output channels, a plurality of signals each representing a movement component and reducing, for each of the output channels, signals based on reflected waves from an unmoving reflector by using the plurality of received signals;
performing adaptive beam forming (S103) by using the plurality of signals each representing the movement component; and
acquiring the movement information (S104) of the object by using a plurality of output signals obtained by the adaptive beam forming.

10. The subject information acquisition method according to claim 9, further comprising:
extracting, in extracting the plurality of signals each representing the movement component, for each of the output channels, a plurality of signals each representing the time varying component between received signals obtained by transmitting the acoustic wave pulse a plurality of times as the signals each representing the movement component.

11. The subject information acquisition method according to claim 10, further comprising extracting, in extracting the plurality of signals each representing the movement component, the plurality of signals each representing the time varying component between the plurality of received signals, by obtaining, for each of the output channels, differences between the plurality of received signals obtained by transmitting the acoustic wave pulse a plurality of times.

12. The subject information acquisition method according to claim 10, further comprising extracting, in extracting the plurality of signals each representing the movement component, the plurality of signals each representing the movement component in the received signals by performing, for each of the output channels, a quadrature detection on the plurality of received signals obtained by transmitting the acoustic wave pulse for a plurality of times, and performing low-pass filter processing and high-pass filter processing.

13. The subject information acquisition method according to any one of claims 9 to 12, further comprising extracting, in extracting the plurality of signals each representing the movement component, a signal based on a reflected wave from a reflector moving in the subject at a higher velocity than a predetermined velocity as the signal representing the movement component.

14. The subject information acquisition method according to any one of claims 9 to 13, further comprising performing, as the adaptive beam forming, processing of minimizing power with a sensitivity related to a target direction fixed, by using the plurality of signals each representing the movement component is performed.

15. The subject information acquisition method according to any one of claims 9 to 14 further comprising:

acquiring an acoustic characteristic distribution reflecting a difference in an acoustic impedance in the subject; and
generating display data by using the movement information and the acoustic characteristic distribution.

16. The subject information acquisition method according to claim 11, wherein the method further performs a compensation processing for reducing an effect of taking the difference.

17. A program for causing a computer to execute the subject information acquisition method according to any one of claims 9 to 16.


**Patentansprüche**

1. Subjektinformationserfassungsvorrichtung, die konfiguriert ist, Bewegungsinformation eines Objekts in einem Subjekt zu erfassen, wobei die Subjektinformationserfassungsvorrichtung umfasst:

mehrere Wandler (002), die konfiguriert sind, einen akustischen Wellenimpuls an das Subjekt zu übertragen, von mehreren Positionen im Subjekt reflektierte Wellen zu empfangen, wobei jeder der mehreren Wandler an einen entsprechenden Ausgangskanal gekoppelt ist, und die reflektierten Wellen jeweils in zeitseriell empfangene Signale umzuwandeln; und
eine Verarbeitungseinheit (006, 007, 008), die konfiguriert ist, die Bewegungsinformation des Objekts durch Verwenden mehrerer der empfangenen Signale zu erfassen, die von den mehreren Wandlern über die entsprechenden Ausgangskanäle ausgegeben werden,
**dadurch gekennzeichnet, dass** die Verarbeitungseinheit für jeden der Ausgangskanäle mehrere Signale extrahiert, die jeweils eine Bewegungskomponente darstellen, und unter Verwendung der mehreren empfangenen Signale basierend auf von einem unbewegten Reflektor reflektierten Wellen für jeden der Ausgangskanäle Signale reduziert, adaptive Strahlerzeugung unter Verwendung der mehreren Signale durchführt, welche jeweils die Bewegungskomponente darstellen, und die Bewegungsinformation des Objekts unter Verwendung mehrerer durch die adaptive Strahlerzeugung erhaltener Ausgabesignale erfasst.

2. Subjektinformationserfassungsvorrichtung nach Anspruch 1, wobei die Verarbeitungseinheit für jeden der Ausgangskanäle als die jeweils die Bewegungskomponente darstellende Signale mehrere Signale extrahiert, die jeweils eine zeitveränderliche Komponente zwischen empfangenen Signalen darstellen, die erhalten werden durch mehrmaliges Übertragen des akustischen Wellenimpulses.

3. Subjektinformationserfassungsvorrichtung nach Anspruch 2, wobei die Verarbeitungseinheit die mehreren Signale, die jeweils die zeitveränderliche Komponente zwischen den mehreren empfangenen Signalen darstellen, extrahiert, indem für jeden der Ausgangskanäle eine Differenz zwischen den mehreren empfangenen Signalen erhalten wird, die durch mehrmaliges Übertragen der akustischen Wellenimpulsübertragung erhalten werden.

4. Subjektinformationserfassungsvorrichtung nach Anspruch 2, wobei die Verarbeitungseinheit die mehreren Signale, die jeweils die Bewegungskomponente darstellen, extrahiert, indem für jeden der Ausgangskanäle Tiefpassfilterverarbeitung und Hochpassfilterverarbeitung durchgeführt wird, nachdem an den mehreren durch mehrmaliges Übertragen des akustischen Wellenpulses erhaltenen empfangenen Signalen eine Quadraturdetektion durchgeführt wurde.

5. Subjektinformationserfassungsvorrichtung nach einem der Ansprüche 1 bis 4, wobei die Verarbeitungseinheit als das Signal, das die Bewegungskomponente darstellt, ein Signal extrahiert, und zwar basierend auf einer reflektierten Welle von einem Reflektor, der sich im Subjekt mit höherer Geschwindigkeit als einer vorbestimmten Geschwindigkeit bewegt.

6. Subjektinformationserfassungsvorrichtung nach einem der Ansprüche 1 bis 5, wobei die Verarbeitungseinheit als die adaptive Strahlerzeugung Verarbeitung des Minimierens elektrischer Leistung mit einer Empfindlichkeit bezüglich einer fixierten Zielrichtung durchführt, indem die mehreren Signale verwendet werden, die jeweils die Bewegungskomponente darstellen.

7. Subjektinformationserfassungsvorrichtung nach einem der Ansprüche 1 bis 6, wobei die Verarbeitungseinheit ferner eine Verteilung einer akustischen Charakteristik erfasst, die einen Unterschied einer akustischen Impedanz im Subjekt wiederspiegelt, und Anzeigedaten erzeugt, indem die Bewegungsinformation und die Verteilung der akustischen Charakteristik genutzt werden.

8. Subjektinformationserfassungsvorrichtung nach Anspruch 3, wobei die Verarbeitungseinheit ferner eine Kompensationsverarbeitung durchführt, um einen Differenzbildungseffekt zu reduzieren.

9. Subjektinformationserfassungsverfahren des Erfassens von Bewegungsinformation eines Objekts in einem Subjekt durch Verwenden mehrerer zeitseriell empfangener Signale, die von mehreren Wandlern ausgegeben werden, welche einen akustischen Wellenimpuls an das Subjekt übertragen und von mehreren Positionen im Subjekt reflektierte Wellen empfangen, wobei jeder der mehreren Wandler an einen entsprechenden Ausgangskanal gekoppelt ist, wobei das Subjektinformationserfassungsverfahren umfasst:

Extrahieren (S102) mehrerer Signale für jeden der Ausgangskanäle, die jeweils eine Bewegungskomponente darstellen, und Reduzieren von Signalen für jeden der Ausgangskanäle basierend auf reflektierten Wellen von einem unbewegten Reflektor durch Verwenden der mehreren empfangenen Signale;
Durchführen von adaptiver Strahlerzeugung (S103) durch Verwenden der mehreren Signale, welche jeweils die Bewegungskomponente darstellen; und
Erfassen der Bewegungsinformation (S104) des Objekts durch Verwenden mehrerer durch die adaptive Strahlerzeugung erhaltener Ausgabesignale.

10. Subjektinformationserfassungsverfahren nach Anspruch 9, ferner umfassend:
Extrahieren mehrerer Signale, die jeweils die zeitveränderliche Komponente zwischen empfangenen Signalen darstellen, die durch mehrmaliges Übertragen des akustischen Wellenpulses erhalten werden, für jeden der Ausgangskanäle beim Extrahieren der mehreren Signale, die jeweils die Bewegungskomponente darstellen.

11. Subjektinformationserfassungsverfahren nach Anspruch 10, ferner umfassend Extrahieren der mehreren Signale, die jeweils die zeitveränderliche Komponente zwischen den mehreren empfangenen Signalen darstellen, indem für jeden der Ausgangskanäle Differenzen zwischen den mehreren empfangenen Signalen erfasst werden, die durch mehrmaliges Übertragen der akustischen Wellenimpulse erhalten werden.

12. Subjektinformationserfassungsverfahren nach Anspruch 10, ferner umfassend, und zwar beim Extrahieren der mehreren Signale, die jeweils die Bewegungskomponente in den empfangenen Signalen darstellen, Extrahieren der mehreren Signale, die jeweils die Bewegungskomponente in den empfangenen Signalen darstellen, indem für jeden der Ausgangskanäle eine Quadraturdetektion an den mehreren empfangenen Signalen durchgeführt wird, die erhalten werden durch mehrmaliges Übertragen des akustischen Wellenimpulses, sowie Durchführen von Tiefpassfilterverarbeitung und Hochpassfilterverarbeitung.

**13.** Subjektinformationserfassungsverfahren nach einem der Ansprüche 9 bis 12, ferner umfassend, und zwar beim Extrahieren eines Signals als das die Bewegungskomponente darstellende Signal, Extrahieren eines Signals als das die Bewegungskomponente darstellende Signal basierend auf einer Welle, die von einem Reflektor reflektiert wird, der sich im Subjekt mit höherer Geschwindigkeit als einer vorbestimmten Geschwindigkeit bewegt, als das die Bewegungskomponente darstellende Signal.

**14.** Subjektinformationserfassungsverfahren nach einem der Ansprüche 9 bis 13, ferner umfassend, und zwar als die adaptive Strahlerzeugung, Durchführen von Verarbeitung des Minimierens elektrischer Leistung mit Empfindlichkeit bezüglich einer fixierten Zielrichtung, indem die mehreren Signale verwendet werden, die jeweils die Bewegungskomponente darstellen.

**15.** Subjektinformationserfassungsverfahren nach einem der Ansprüche 9 bis 14, ferner umfassend:

Erfassen einer Verteilung einer akustischen Charakteristik, die einen Unterschied einer akustischen Impedanz im Subjekt reflektiert, und
Erzeugen von Anzeigedaten durch Verwenden der Bewegungsinformation und der Verteilung der akustischen Charakteristik.

**16.** Subjektinformationserfassungsverfahren nach Anspruch 11, wobei das Verfahren ferner eine Kompensationsverarbeitung durchführt, um einen Differenzbildungseffekt zu reduzieren.

**17.** Programm zum Veranlassen eines Computers, das Subjektinformationserfassungsverfahren nach einem der Ansprüche 9 bis 16 auszuführen.

## Revendications

**1.** Appareil d'acquisition d'informations de sujet configuré pour acquérir des informations de mouvement d'un objet dans un sujet, l'appareil d'acquisition d'informations de sujet comprenant :

une pluralité de transducteurs (002) configurés pour transmettre une impulsion d'onde acoustique en direction du sujet, pour recevoir des ondes réfléchies, réfléchies à partir d'une pluralité de positions dans le sujet, chacun de la pluralité de transducteurs étant couplé à un canal de sortie correspondant, et pour convertir respectivement les ondes réfléchies en signaux de série chronologique reçus ; et
une unité de traitement (006, 007, 008) configurée pour acquérir les informations de mouvement de l'objet au moyen d'une pluralité des signaux reçus délivrés à partir de la pluralité de transducteurs par le biais des canaux de sortie correspondants,
**caractérisé en ce que** l'unité de traitement extrait, pour chacun des canaux de sortie, une pluralité de signaux représentant chacun une composante de mouvement, et réduit, pour chacun des canaux de sortie, les signaux basés sur des ondes réfléchies à partir d'un réflecteur immobile au moyen de la pluralité de signaux reçus, exécute une formation de faisceau adaptative au moyen de la pluralité de signaux représentant chacun la composante de mouvement, et acquiert les informations de mouvement de l'objet au moyen d'une pluralité de signaux de sortie obtenus par la formation de faisceau adaptative.

**2.** Appareil d'acquisition d'informations de sujet selon la revendication 1, dans lequel l'unité de traitement extrait, pour chacun des canaux de sortie, une pluralité de signaux représentant chacun une composante qui varie dans le temps entre les signaux reçus obtenus par une transmission, une pluralité de fois, de l'impulsion d'onde acoustique, en tant que signaux représentant chacun la composante de mouvement.

**3.** Appareil d'acquisition d'informations de sujet selon la revendication 2, dans lequel l'unité de traitement extrait la pluralité de signaux représentant chacun la composante qui varie dans le temps entre la pluralité de signaux reçus, en obtenant, pour chacun des canaux de sortie, une différence entre la pluralité de signaux reçus obtenus par une transmission, une pluralité de fois, de l'impulsion d'onde acoustique.

**4.** Appareil d'acquisition d'informations de sujet selon la revendication 2, dans lequel l'unité de traitement extrait la pluralité de signaux représentant chacun la composante de mouvement par une exécution, pour chacun des canaux de sortie, d'un traitement de filtrage passe-bas et d'un traitement de filtrage passe-haut, après l'exécution d'une détection de quadrature par rapport à la pluralité de signaux reçus obtenus par une transmission, une pluralité de fois, de l'impulsion d'onde acoustique.

5. Appareil d'acquisition d'informations de sujet selon l'une quelconque des revendications 1 à 4, dans lequel l'unité de traitement extrait, en tant que signal représentant la composante de mouvement, un signal basé sur une onde réfléchie à partir d'un réflecteur en mouvement dans le sujet à une vitesse supérieure à une vitesse prédéterminée.

6. Appareil d'acquisition d'informations de sujet selon l'une quelconque des revendications 1 à 5, dans lequel l'unité de traitement exécute, en tant que formation de faisceau adaptative, un traitement d'abaissement au minimum d'énergie électrique avec une sensibilité fixe associée à une direction de cible, au moyen de la pluralité de signaux représentant chacun la composante de mouvement.

7. Appareil d'acquisition d'informations de sujet selon l'une quelconque des revendications 1 à 6, dans lequel l'unité de traitement acquiert en outre une distribution de caractéristiques acoustiques reflétant une différence d'impédance acoustique dans le sujet, et génère des données d'affichage au moyen des informations de mouvement et de la distribution de caractéristiques acoustiques.

8. Appareil d'acquisition d'informations de sujet selon la revendication 3, dans lequel l'unité de traitement exécute en outre un traitement de compensation pour réduire un effet de considération de la différence.

9. Procédé d'acquisition d'informations de sujet consistant à acquérir des informations de mouvement d'un objet dans un sujet au moyen d'une pluralité de signaux de série chronologique reçus, délivrés à partir d'une pluralité de transducteurs qui transmettent une impulsion d'onde acoustique en direction du sujet et qui reçoivent des ondes réfléchies, réfléchies à partir d'une pluralité de positions dans le sujet, chacun de la pluralité de transducteurs étant couplé à un canal de sortie correspondant, le procédé d'acquisition d'informations de sujet comprenant les étapes consistant à :

extraire (S102), pour chacun des canaux de sortie, une pluralité de signaux représentant chacun une composante de mouvement et réduire, pour chacun des canaux de sortie, les signaux basés sur des ondes réfléchies à partir d'un réflecteur immobile au moyen de la pluralité de signaux reçus ;
exécuter une formation de faisceau adaptative (S103) au moyen de la pluralité de signaux représentant chacun la composante de mouvement ; et
acquérir les informations de mouvement (S104) de l'objet au moyen d'une pluralité de signaux de sortie obtenus par la formation de faisceau adaptative.

10. Procédé d'acquisition d'informations de sujet selon la revendication 9, comprenant en outre l'étape consistant à :
extraire, lors de l'extraction de la pluralité de signaux représentant chacun la composante de mouvement, pour chacun des canaux de sortie, une pluralité de signaux représentant chacun la composante qui varie dans le temps entre des signaux reçus obtenus par une transmission, une pluralité de fois, de l'impulsion d'onde acoustique, en tant que signaux représentant chacun la composante de mouvement.

11. Procédé d'acquisition d'informations de sujet selon la revendication 10, comprenant en outre l'étape consistant à extraire, lors de l'extraction de la pluralité de signaux représentant chacun la composante de mouvement, la pluralité de signaux représentant chacun la composante qui varie dans le temps entre la pluralité de signaux reçus, en obtenant, pour chacun des canaux de sortie, des différences entre la pluralité de signaux reçus obtenus par une transmission, une pluralité de fois, de l'impulsion d'onde acoustique.

12. Procédé d'acquisition d'informations de sujet selon la revendication 10, comprenant en outre les étapes consistant à extraire, lors de l'extraction de la pluralité de signaux représentant chacun la composante de mouvement, la pluralité de signaux représentant chacun la composante de mouvement dans les signaux reçus par une exécution, pour chacun des canaux de sortie, d'une détection de quadrature par rapport à la pluralité de signaux reçus obtenus par une transmission, une pluralité de fois, de l'impulsion d'onde acoustique, et à exécuter un traitement de filtrage passe-bas et un traitement de filtrage passe-haut.

13. Procédé d'acquisition d'informations de sujet selon l'une quelconque des revendications 9 à 12, comprenant en outre l'étape consistant à extraire, lors de l'extraction de la pluralité de signaux représentant chacun la composante de mouvement, un signal basé sur une onde réfléchie à partir d'un réflecteur en mouvement dans le sujet à une vitesse supérieure à une vitesse prédéterminée en tant que signal représentant la composante de mouvement.

**14.** Procédé d'acquisition d'informations de sujet selon l'une quelconque des revendications 9 à 13, comprenant en outre l'étape consistant à exécuter, en tant que formation de faisceau adaptative, un traitement de diminution au minimum d'énergie avec une sensibilité fixe associée à une direction de cible, au moyen de la pluralité de signaux représentant chacun la composante de mouvement.

**15.** Procédé d'acquisition d'informations de sujet selon l'une quelconque des revendications 9 à 14, comprenant en outre les étapes consistant à :

acquérir une distribution de caractéristiques acoustiques reflétant une différence d'impédance acoustique dans le sujet ; et
générer des données d'affichage au moyen des informations de mouvement et de la distribution de caractéristiques acoustiques.

**16.** Procédé d'acquisition d'informations de sujet selon la revendication 11, dans lequel le procédé consiste en outre à exécuter un traitement de compensation pour réduire un effet de considération de la différence.

**17.** Programme destiné à amener un ordinateur à exécuter le procédé d'acquisition d'informations de sujet selon l'une quelconque des revendications 9 à 16.

## FIG. 1

EP 2 956 798 B1

# FIG. 2

```
        ┌─────────────┐
        │    START    │
        └──────┬──────┘
               │
               ▼
     ┌─────────────────────┐
     │ INPUT RECEIVED SIGNAL│ ~ S101
     └──────────┬──────────┘
               │
               ▼
     ┌─────────────────────┐
     │ PERFORM DIFFERENTIAL │ ~ S102
     │ PROCESSING          │
     └──────────┬──────────┘
               │
               ▼
     ┌─────────────────────┐
     │ PERFORM ADAPTIVE    │ ~ S103
     │ SIGNAL PROCESSING   │
     └──────────┬──────────┘
               │
               ▼
     ┌─────────────────────┐
     │ CALCULATE FLOW VELOCITY│ ~ S104
     └──────────┬──────────┘
               │
               ▼
        ┌─────────────┐
        │     END     │
        └─────────────┘
```

FIG. 3A

xk, l [s] ——————————→(+ Σ) ——→ dk, l [s]

Delay
T

FIG. 3B

xk, l [s] ——————→(+ Σ)————————→(+ Σ) ——→ dk, l [s]

Delay
T

Delay
T

FIG. 4

# FIG. 5

FIG. 6

## FIG. 7

TRANSMITTING CIRCUIT SYSTEM — 003

DELAY-AND-SUM BLOCK — 612

ENVELOPE DETECTION BLOCK — 613

RECEIVING CIRCUIT SYSTEM — 005

QUADRATURE DETECTION BLOCK — 606

WALL FILTER BLOCK — 611

ADAPTIVE SIGNAL PROCESSING BLOCK — 607

FLOW VELOCITY CALCULATION BLOCK — 608

DISPLAY PROCESSING BLOCK — 609

DISPLAY SYSTEM — 610

SYSTEM CONTROL UNIT — 004

001

002

000

EP 2 956 798 B1

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5718229 A

- JP 1153144 A

**Non-patent literature cited in the description**

- Blood velocity estimation using ultrasound and spectral iterative adaptive approaches. **ERIK GUDMUND-SON et al.** SIGNAL PROCESSING. ELSEVIER SCIENCE PUBLISHERS, 26 December 2010, vol. 91, 1275-1283

- Benefits of minimum-variance beamforming in medical ultrasound imaging. **J-F SYNNEVAG et al.** IEEE TRANSACTIONS ON ULTRASONICS, FERROELECTRICS AND FREQUENCY CONTROL. IEEE, 01 September 2009, vol. 56, 1868-1879